# EUROPEAN PATENT APPLICATION

(11) **EP 1 262 157 A1**
(43) Date of publication of application: **04.12.2002**
(21) Application number: 01810520.5
(22) Date of filing: 29.05.2001
(51) Int. Cl.: A61F 9/013, A61F 9/007, A61B 17/32

(54) **Scalpel for ophtalmology surgery**

(71) Applicant: Anton Meyer & Co.AG, 2560 Nidau (CH)
(72) Inventor: Meyer, Rolf, 2562 Port (CH); Meyer, Thomas, 2563 Ipsach (CH)
(74) Representative: Clerc, Natalia

(57) **Abstract**

A scalpel for ophthalmology surgery comprises a handle (1) and a blade (2) mounted in the handle (1). The blade (2) is made of titanium or a titanium alloy. This blade (2) incorporates a good combination of the advantages of the known steel and diamond blades, rendering the blade and its handle inexpensive but representing a useful alternative to the diamond blade.

## Description

### Field of the invention

The invention relates to a scalpel for ophthalmology surgery in accordance with the preamble of claim 1, a blade system in accordance with the preamble of claim 6 and a blade in accordance with the preamble of claim 9.

### Description of the related art

In the state of the art, two different kind of scalpel blades are used in ophthalmology surgery: blades made of stainless steel and blades made of diamonds.

Blades made of stainless steel have the advantage, that they are quite inexpensive. Furthermore, their resistance to rupture is quite good. However, these blades have several disadvantages: The blades tend to rust. Furthermore, they become dull when sterilized. This tendency even increases with higher sterilization temperature. Since nowadays in view of the mad cow disease, it is important to use quite high sterilization temperatures, i.e. over 140°C. Stainless steel blades can therefore hardly be used anymore. In addition, their surfaces are quite rough. Since they usually can only be used one single time, the costs for this blade has to be paid by one patient only.

Diamond blades however do overcome the disadvantages of the stainless steel blades. They do not rust or become dull. They can be reused, dividing its costs among several patients. They also can be re-sharpened several times. Their surfaces are smoother and furthermore, they have an excellent abrasive hardness, enabling to cut the cornea without inducing remarkable pressure, therefore reducing the risk of astigmatism. The main disadvantages of these blades are the high prize, often leading the customer to buy the cheaper steel blade, and their poor resistance to rupture, especially when handled without care. To prevent breakage of the diamond blades, they are usually protected by a blade guard or they can be moved into a handle of the scalpel when not in use. However, such scalpels are more complicated and therefore more expensive.

### Summary of the invention

It is therefore an object of the invention to provide a scalpel, a blade system and a blade combining the advantages of the steel and diamond blades, but avoiding as many of the above mentioned disadvantages, especially being less expensive in production than the diamond blade.

The object is achieved by a scalpel, a blade system and a blade with the features of claim 1, claim 6 and claim 9 respectively.

In accordance with the present invention, the blade is made of titanium or a titanium alloy. This blade has the advantages, that it does not rust, that it does not become dull when sterilized, that it has a smooth surface, that it is unaffected by changes of temperature and that it is well accepted by the human body. In addition, its production is cheaper than the one for diamond blades. Furthermore, the material the blade is made of is not magnetic. This is important in the operation theatre, where items should not be attracting or been attracted by other items lying on the same table.

Since this blade is not susceptible to break, the scalpel can have a simpler shape, reducing the costs as well.

In one embodiment of the invention, the blade is disposable, intended to be used only one single time. In another embodiment, the blade is reusable.

Other preferred embodiments are mentioned in the dependent claims.

### Brief description of the drawings

In the following, preferred embodiments of the invention are explained with reference to the drawings. The drawings show
- figure 1: a view of a scalpel;
- figure 2: a view of a blade system;
- figure 3a: a view of the blade in the embodiment according to figure 2;
- figure 3b: a side view of the blade of figure 3a and
- figure 4: a view of a blade in a second embodiment of the invention.

### Description of the preferred embodiments

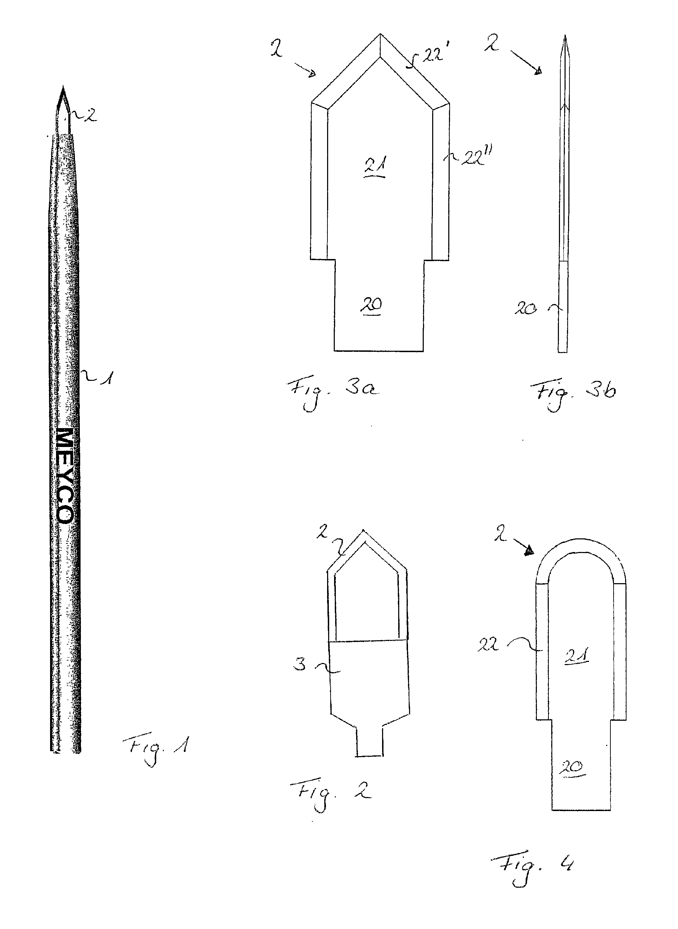

The scalpel according to figure 1 has a handle 1, in which a blade 2 is mounted. The blade 2 is made of titanium or a titanium alloy. Preferably, it is made of titanium alloy ASTM grade 2, ASTM grade 5 or another titanium alloy being suitable to grind thin blades with sharp cutting edges, a typical blade thickness ranging between 0.1 and 0.2 mm.

In the embodiment shown in figures 1, 3a and 3b the blade 2 is mounted directly in the handle 1. In this embodiment, the blade 2 is fixed non-removable in the handle 1, for example with an adhesive. In another embodiment, the blade 2 is removable.

The shape of the blade 2 depends on the kind of surgery, it is intended to be used for. Normally, it has a blade head 21 with one or more cutting edges 22', 22" and a blade heel 20 for mounting the blade 2 directly into the handle 1 or into a holder 3, the holder 3 being removably fixed in the handle 1 by known means. Preferably, the holder 3 is made of titanium or a titanium alloy too. In one embodiment, the holder 3 and the blade 2 are made in one single piece. In another embodiment, the holder 3 and the blade 2 are separate pieces. Such a holder 3 with a mounted blade is shown in figure 2.

In this embodiment, the blade 2 has four cutting edges, two 22' of them forming the sides of an angle, the other two 22" being parallel to each other and forming a prolongation of the angle sides.
Figure 4 shows another embodiment of the blade 2. Here, the blade 2 has curved cutting edges 22.

The shape of the blade 2 depends on the kind of surgery they are to be used for. These titanium blades 2 can be used for any kind of eye surgery where the use of steel and/or diamond blades is known, for example for cataract surgery (such as clear cornea incision, tunnel incision, side-port incision, scleral no-stitch incision or phaco incision), for glaucoma surgery (such as trabeculectomy or deep sclerectomy) or for refractive surgery (such as astigmatic correction, ICL implantation or radial keratotomy).

The blade 2 in accordance with the invention incorporates a good combination of the advantages of the known steel and diamond blades, rendering the blade and its handle inexpensive but representing a useful alternative to the diamond blade.

### Reference numbers

- 1: handle
- 2: blade
- 20: blade heel
- 21: blade head
- 22: cutting edge
- 22', 22": cutting edge
- 3: holder

## Claims

1. A scalpel for ophthalmology surgery, comprising a handle (1) and a blade (2) mounted in the handle (1), **characterized in that** the blade (2) is made of titanium or a titanium alloy.

2. The scalpel, as claimed in claim 1, wherein the blade (2) is made of titanium alloy ASTM grade 2 or ASTM grade 5.

3. The scalpel, as claimed in claim 1, wherein the blade (2) is mounted in a holder (3), the holder (3) being mounted in the handle (1).

4. The scalpel, as claimed in claim 2, wherein the holder (3) is removably mounted in the handle (1).

5. The scalpel, as claimed in claim 2, wherein the blade (2) is non-removably mounted in the holder (3).

6. A blade system to be used in a scalpel for ophthalmology surgery with a holder (3) and a blade (2), **characterized in that** the blade (2) is made of titanium or a titanium alloy.

7. The blade system, as claimed in claim 6, wherein the blade (2) is made of titanium alloy ASTM grade 2 or ASTM grade 5.

8. The blade system, as claimed in claim 6, wherein the holder (3) is made of titanium or a titanium alloy.

9. A blade (2) to be used in a scalpel for ophthalmology surgery, **characterized in that** the blade (2) is made of titanium or a titanium alloy.
